# EUROPÄISCHE PATENTSCHRIFT

(11) **EP 0 396 860 B1**
(45) Veröffentlichungstag und Bekanntmachung des Hinweises auf die Patenterteilung: **30.11.1994**
(21) Anmeldenummer: 90103413.2
(22) Anmeldetag: 22.02.1990
(51) Int. Cl.: A61B 8/08, A61B 17/22

(54) **Ortungskinematik für einen Lithotripter**
Cinematic detector for a lithotripter
Détection cinématique pour un lithotripteur

(30) Priorität: 11.05.1989 DE 3915384
(43) Veröffentlichungstag der Anmeldung: 14.11.1990
(73) Patentinhaber: DORNIER MEDIZINTECHNIK GMBH, D-82101 Germering (DE)
(72) Erfinder: Viebach, Thomas, Dipl.-Ing., D-8081 Pischertshofen (DE); Buchbauer, Peter, Dipl.-Ing., D-8046 Garching (DE); Herrmann, Bernhard, Dipl. Ing., D - 8034 Germering (DE)
(74) Vertreter: Zwergel, Wilhelm

(56) Entgegenhaltungen:
- EP-A- 0 169 311
- EP-A- 0 288 698
- DE-U- 8 800 985

## Beschreibung

Die Erfindung betrifft eine Vorrichtung zur Ortung von Konkrementen im Körper eines Patienten an einem Lithotripter mit einem Ultraschall-Transducer nach dem Oberbegriff des Anspruchs 1.

Bekannt sind Lithotripter mit raumfesten Stosswellenquellen, bei denen die Patientenliege solange verschoben wird, bis der zu behandelnde Bereich im Patienten im Fokusbereich der Stosswellenquelle liegt.
Bei anderen Modellen sind sowohl die Stosswellenquelle - die sich z.B. an einem Therapiekopf mit Mitteln zum Fokussieren und Einleiten der Stosswelle befindet - als auch die Patientenliege beweglich.

Die DE-PS 34 27 001 beschreibt einen zwangsgeführten Ultraschall-Transducer, der stets auf einen Punkt gerichtet ist. Die dort offenbarte Mechanik ist jedoch relativ groß und unhandlich.

Aufgabe der Erfindung ist es, an einer solchen Vorrichtung, insbesondere an einem Lithotripter, eine Ortungskinematik vorzuschlagen, mit der die Ortung und die Positionierung vereinfacht wird.

Diese Aufgabe wird erfindungsgemäß gelöst von einer Vorrichtung mit den Merkmalen des Anspruchs 1.

Ausführungen der Erfindung sind Gegenstände von abhängigen Ansprüchen.

Erfindungsgemäß ist der Ultraschall-Transducer so am Therapiekopf befestigt, daß er stets genau auf den Punkt "zielt", auf den die Stosswellen fokussiert werden. Eine Korrelation, wie sie z.B. in der DE-PS 34 27 001 noch zwischen dem Isozentrum des Transducers und dem Fokuspunkt der Stoßwellenquelle notwendig war, ist jetzt nicht mehr notwendig. Der Arzt ist frei in der Wahl der Beobachtungsfenster oder der Behandlungsfenster und kann trotzdem schnell den Stein finden und den Patienten relativ zum Therapiekopf so positionieren, daß eine Steinzertrümmerung erfolgen kann.

In einer Ausführung ist der Transducer an einem Ring befestigt, der den Therapiekopf umfasst und dessen Drehachse mit der Achse des Therapiekopfes zusammenfällt. Der Transducer kann z.B. von einer Kinematik geführt sein, die drei Hebel enthält, wobei der erste Hebel und der dritte Hebel so zwangsgeführt sind, daß sie stets parallel zueinander verlaufen.
Bewegungsmöglichkeiten des Transducers um seine eigene Längsachse oder eine Verschiebung in seiner Längsachse sind ebenso möglich und geben dem Arzt weitere Freiheiten in der Wahl des Fensters.

Die Erfindung wird anhand von drei Figuren näher erläutert.

Es zeigen:
- Figur 1: die Bewegungsmöglichkeiten der erfindungsgemäßen Ortungskinematik,
- Figur 2: eine Ausführung einer Ortungskinematik,
- Figur 3: drei Ultraschallbilder, anhand derer ein Ortungsvorgang erläutert wird.

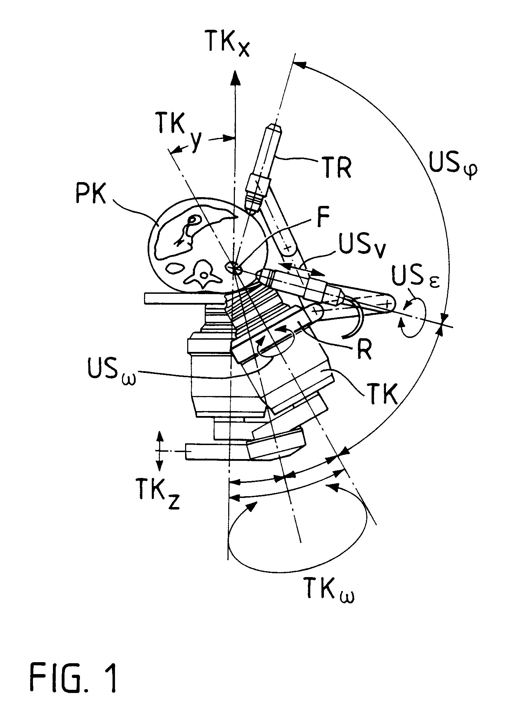

Figur 1 zeigt einen Patientenkörper PK, in dem sich eine Niere mit Nierenstein im Punkt F befindet. Unterhalb des Patientenkörpers PK befindet sich der Therapiekopf TK, der Vorrichtungen zum Erzeugen, Fokussieren und Einleiten von Stosswellen in den Patientenkörper PK enthält. Am Therapiekopf TK ist der Ultraschall-Transducer TR beweglich befestigt. In dieser Ausführung ist der Transducer TR über einen Ring R am Therapiekopf TK befestigt. Der Ring R ist um den Therapiekopf TR drehbar befestigt. Am Ring R sind drei zweiarmige Hebel angeordnet, die den Transducer TR führen und die bei Verschwenkungen des Transducer TR um den Winkel US_{φ} den Transducer TR immer auf den Fokuspunkt F zielen lassen. Weiter eingezeichnet sind die Bewegungsmöglichkeiten USᵥ des Ultraschall-Transducer TR durch Verschieben längs seiner Längsachse, US_{ε}, die Verdrehung des Transducers TR um seine Längsachse und Us_{ω}, die Verdrehung des Transducers um die Längsachse des Therapiekopfes TK. Als Bewegungsmöglichkeiten für den Therapiekopf sind eingezeichnet:
- TK_{z} die Verschiebung des Therapiekopfes nach oben und unten,
- TK_{ω} die Verdrehung des Therapiekopfes auf einem Kegelmantel, woraus ein Schwenken des Therapiekopfes gegenüber der x- und y-Achsen um TKₓ und TK_{y} (z.B. um 30°) erfolgt.

In einer Ausführung kann die Bewegung des Transducers TR in Längsachse (USᵥ) elektronisch aufgenommen und die Lage des therapeutischen Fokus F im Ultraschallbild angezeigt werden. Da die Verfahrbewegungen des Transducers TR stets isozentrisch um F verlaufen, ist der Brennpunkt F stets in der Mittellinie des Ultraschallsektorbildes sichtbar.

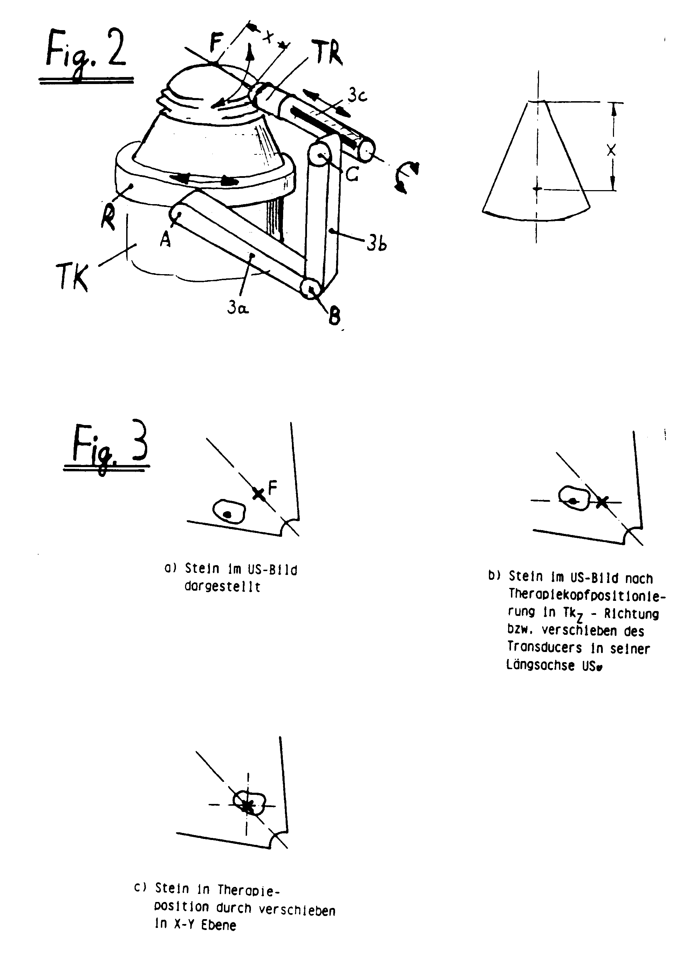

Figur 2 zeigt eine Ausführung der Ortungskinematik mit dem Therapiekopf TK, dem Ring R, den drei zweiarmigen Hebeln mit den Armen 3a, 3b und 3c und dem Transducer TR. Der Ring R ist um den Therapiekopf drehbar. Die drei Hebel haben die Drehachsen A, B und C. Mit den Pfeilen sind die Bewegungsmöglichkeiten des Transducers TR angezeigt:
- Längsverschiebung längs seiner Achse,
- Drehung um seine Längsachse,
- Drehung um die Längsachse des Therapiekopfes
   und
- Verschwenkung um eine durch den Fokus F laufende Achse, die senkrecht zur Achse des Therapiekopfes steht.

Im rechten Teil der Figur 2 ist schematisch ein Ultraschall-Sektor-Scan-Bild gezeigt, in das der Abstand x zum Fokus F eingeblendet ist.

Die Positionierung des Steins erfolgt mit Hilfe des Ultraschall-Transducers TR durch Bewegung des Therapiekopfes TK relativ zum Patienten. Die Bewegungsmöglichkeiten, die dem Transducer TR durch die Mechanik gegeben sind, sind dadurch gekennzeichnet, daß die Mittelachse (Symmetrieachse des Ultraschallbildes) stets durch den Therapiefokus F läuft. Der Abstand x des Transducers TR zum Therapiefokus F ist variabel und wird ständig auf der Mittelachse des Ultraschallbildes angezeigt. Mit dieser Anordnung kann der behandelnde Arzt bei der Ortung des Steines, dessen Positionierung und bei der Beobachtung des Zertrümmerungsvorgangs den Transducer mit größtmöglicher Freiheit am Patienten bewegen (z.B. zum Aufsuchen eines guten Schallfensters), ohne dabei den Bezug zum Therapiefokus F zu verlieren.

Die Bewegungen, die die drei Hebel des Armes 3a, 3b und 3c zueinander in ihren Gelenkpunkten A, B, C ausführen, sind durch Übertragungselemente (Zahnriemen, Zahnräder, Ketten oder Schubstangen, die sich z.B. im Inneren der Gelenke befinden können) miteinander gekoppelt, so daß alle Gelenke stets den gleichen Drehwinkel ausführen. In eine Ebene projiziert bilden die drei Hebel 3a, 3b und 3c zusammen mit der gedachten Verbindungslinie des Gelenkes A mit dem Therapiefokus F ein Parallelogramm.

Der Transducer TR führt dadurch um F analoge Bewegungen aus wie der Hebel 3a um das Gelenk A. Der Hebel 3c ist mit dem Transducer TR bezüglich des Gelenkes C verschiebbar, so daß mit dem Transducer der variable Abstand vom Therapiefokus F (und damit vom Stein) zur Patientenoberfläche ausgeglichen werden kann. Der Transducer TR kann mittels einer Feder an den Patienten angedrückt werden, so daß die Relativbewegungen des Patienten zur Ortungsmechanik stets ausgeglichen werden.
Zur Wahl der Scan-Ebene kann der Transducer TR um seine eigene Achse drehbar sein. Die Ortungsmechanik ist durch Bremsen in jeder Stellung arretierbar.

Figur 3 zeigt ein Ortungsvorgehen anhand von drei Ultraschallbildern.
Teilbild a zeigt das Ultraschallbild, auf dessen Mittelachse sich der Brennpunkt F befindet, und einen Stein, der sich in der Nähe des Brennpunkts F befindet. Die Ultraschall-Schnittebene ist durch die Verdrehung des Transducers TR um seine eigene Achse so gewählt, daß der Stein in dieser Schnittebene liegt.

Teilbild b zeigt nun den Stein im Ultraschallbild nach Therapiekopf-Positionierung der Patientenliege in z-Richtung. Es ist damit gelungen, den Stein auf die Höhe des Fokus F zu bringen.

Teilbild c zeigt nun das Ultraschallbild nach vollendeter Positionierung. Der Stein ist durch Verschieben der Patientenliege in x- und y-Richtung in die Therapieposition gerückt, das heißt er fällt mit dem Fokus F des Therapiekopfes TK zusammen.

## Patentansprüche

1. Vorrichtung zur Ortung von Konkrementen im Körper eines Patienten an einem Lithotripter, wobei die Vorrichtung einen Ultraschall-Transducer (TR) aufweist, der so zwangsgeführt ist, daß seine Mittelachse auf einen bestimmten Punkt (Isozentrum) gerichtet ist, der Therapiekopf (TK) Mittel zum Erzeugen, Fokussieren und Einleiten von Stosswellen in den Patientenkörper (PK) enthält und das Isozentrum des Transducers (TR) mit dem therapeutischen Fokus (F) des Therapiekopfes (TK) zusammenfällt, **dadurch gekennzeichnet**, daß der Transducer (TR) mit einer Kinematik am beweglichen Therapiekopf befestigt ist, die drei Hebel (3a, 3b, 3c) enthält, wobei der erste Hebel (3a) und der dritte Hebel (3c) so zwangsgeführt sind, daß sie stets parallel zueinander verlaufen.

2. Vorrichtung nach Anspruch 1, **dadurch gekennzeichnet,** daß der Transducer (TR) an einem den Therapiekopf (TK) umfassenden Ring (R) befestigt ist, dessen Drehachse mit der Achse des Therapiekopfes (TK) zusammenfällt.

3. Vorrichtung nach einem der vorhergehenden Ansprüche, **dadurch gekennzeichnet,** daß der Transducer (TR) in seiner Längsachse verschieblich ist.

4. Vorrichtung nach einem der vorhergehenden Ansprüche, **dadurch gekennzeichnet,** daß der Transducer (TR) um seine Längsachse drehbar ist.

## Claims

1. Device for locating concrements in the body of a patient on a lithotripter, and that the device comprises an ultrasound transducer (TR) which is guided in such a manner that its centre axis is oriented towards a specified point (iso-centre), that the therapy head (TK) includes means for producing, focussing and introducing shockwaves into the patient's body (PK), and that the iso-centre of the transducer (TR) coincides with the therapeutical focus (F) of the therapy head (TK), **characterised in that** the transducer (TR) is attached to the movable therapy head by a cinematic device comprising three levers (3a, 3b, 3c), of which the first lever (3a) and the third lever (3c) are guided in such a manner that they always extend parallel to each other.

2. Device according to claim 1, **characterised in that** the transducer (TR) is mounted on a ring (R) which surrounds the therapy head (TK) and the rotary axis of which coincides with the axis of the therapy head (TK).

3. Device according to one of the above claims, **characterised in that** the transducer (TR) is displaceable in its longitudinal axis.

4. Device according to one of the above claims, **characterised in that** the transducer (TR) is rotatable around its longitudinal axis.

## Revendications

1. Dispositif de visée (Z) pour le repérage par rayons X dans un appareil destiné au traitement extra-corporel de patients au moyen d'ondes choc, notamment pour un lithotriteur, caractérisé par un support (T) en matériau transparent aux rayons X qui est fixé sur la source d'ondes de choc (SQ) et porte au moins quatre points de référence (R) non transparents aux rayons X, parmi lesquels au moins deux points sont situés sur une première droite (G1) qui passe par le foyer (F) des ondes de choc et au moins deux autres points sont situés sur une deuxième droite (G2) qui passe également par le foyer (F) des ondes de choc.

2. Dispositif de visée (Z) selon la revendication 1, caractérisé par le fait que les deux droites (G1, G2) forment un angle compris entre 15° et 45°, en particulier un angle de 30°.

3. Dispositif de visée (Z) selon la revendication 1 ou 2, caractérisé par le fait que la distance qui sépare deux points de référence (R) sur les deux droites (G1, G2) est comprise entre 5 et 20 cm, en particulier est de 10 cm.

4. Dispositif de visée (Z) selon l'une des revendications précédentes, caractérisé par le fait que les points de référence (R) sont agencés sous forme de figures géométriques, telles que des sphères, des mires et guidons, des carrés (Q), des triangles (DR) ou des cercles (KR), munies ou non chacune d'un réticule et par le fait que deux figures différentes sont disposées chaque fois sur chaque droite (G1, G2).
